# EUROPEAN PATENT APPLICATION

(11) **EP 4 278 901 A1**
(43) Date of publication of application: **22.11.2023**
(21) Application number: 22739415.2
(22) Date of filing: 12.01.2022
(51) Int. Cl.: A23K 10/16, A23K 10/20, A23K 50/80

(54) **COMPOSITION FOR CONTROLLING INTESTINAL BACTERIAL FLORA**

(30) Priority: 12.01.2021 JP 2021002619
(71) Applicant: SUMITOMO CHEMICAL COMPANY, LIMITED, Tokyo 103-6020 (JP); Flying Spark Ltd, Rehovot 7608804 (IL)
(72) Inventor: YAMAMORI, Akihiro, Kawasaki-shi, Kanagawa 212-0014 (JP); SAITO, Koichi, Osaka-shi, Osaka 554-8558 (JP)
(74) Representative: J A Kemp LLP
(86) International application number: PCT/JP2022/000710
(87) International publication number: WO 2022/154010

(57) **Abstract**

Provided is a technique for controlling intestinal bacterial flora. A composition for controlling the proportion of a bacterium in intestinal bacterial flora, the composition comprising an insect material and the bacterium.

## Description

### Technical Field

The present invention relates to a composition for controlling intestinal bacterial flora, and the like.

### Background Art

As the world's population grows, the demand for food production, such as aquaculture and animal husbandry, increases, and further efficiency is required. For example, for the first time in human history, the catch of farmed fish is expected to exceed the catch of wild fish within two years. However, in aquaculture and animal husbandry, disease caused by pathogens has become a problem, especially in aquatic animals. For example, in salmon farming, the fatality rate due to disease is 20%, and in tilapia, the fatality rate ranges from 40 to 80%.

Vaccination, administration of antibiotics, and other measures have been taken as measures against disease. However, vaccination is only effective against diseases for which vaccines have been developed, and there is also the risk of emergence of mutant strains. In addition, administration of antibiotics has the risk of developing resistant bacteria, and there is also the possibility that resistant bacteria will infect humans.

### Citation List

### Non-patent Literature

NPL 1: Aquaculture, Volume 500, 1 February 2019, Pages 485-491

### Summary of Invention

### Technical Problem

A huge number of bacteria live in the intestine and form intestinal bacterial flora. Intestinal bacterial flora has been found to be involved in various diseases. Therefore, controlling intestinal bacterial flora is considered to, for example, increase the intestinal occupancy of useful diseaseresistant bacteria (e.g., lactic acid bacteria and *Bacillus subtilis*), whereby disease resistance can be imparted.

NPL 1 has reported that the composition of intestinal bacterial flora changed due to feeding fish insects. However, this technique cannot predict how the composition of intestinal bacterial flora will change, and cannot control the intended useful bacteria.

An object of the present disclosure is to provide a technique for controlling intestinal bacterial flora.

### Solution to Problem

The present inventor conducted extensive research, and found that it is possible to control the proportion of a bacterium in intestinal bacterial flora by feeding an insect material and the bacterium. Upon further research based on this finding, the present inventor has completed the present invention. Specifically, the present disclosure includes the following embodiments.

1. A composition for controlling the proportion of a bacterium in intestinal bacterial flora, the composition comprising an insect material and the bacterium.
2. The composition according to Item 1, wherein the bacterium is at least one member selected from the group consisting of bacteria of Firmicutes, bacteria of Bacteroidetes, bacteria of Proteobacteria, and bacteria of Actinobacteria.
3. The composition according to Item 1 or 2, wherein the insect material is a material derived from an insect belonging to Diptera.
4. The composition according to any one of Items 1 to 3, wherein the insect material is at least one member selected from the group consisting of insect extracts and insect homogenates.
5. The composition according to any one of Items 1 to 4, wherein the insect material is a material derived from at least one member selected from the group consisting of eggs, larvae, prepupae, pupae, and adult insects.
6. The composition according to any one of Items 1 to 5, wherein the intestinal bacterial flora is intestinal bacterial flora of an aquatic animal.
7. The composition according to Item 6, wherein the aquatic animal is a fish.
8. The composition according to any one of Items 1 to 7, wherein the content of the insect material is 1 to 50 mass%.
9. The composition according to any one of Items 1 to 8, which is a feed additive or a feed.
10. A method for producing an animal in which the proportion of a bacterium in intestinal bacterial flora is controlled, the method comprising feeding the animal the composition of any one of Items 1 to 9.
11. A method for controlling the proportion of a bacterium in intestinal bacterial flora of an animal, the method comprising feeding the animal the composition of any one of Items 1 to 9.
12. Use of an insect material and a bacterium for producing a composition for controlling the proportion of the bacterium in intestinal bacterial flora.
13. Use of a composition comprising an insect material and a bacterium for controlling the proportion of the bacterium in intestinal bacterial flora.
14. A composition comprising an insect material and a bacterium for use in control of the proportion of the bacterium in intestinal bacterial flora.

### Advantageous Effects of Invention

According to the present disclosure, it is possible to provide a composition for controlling the proportion of a bacterium in intestinal bacterial flora, the composition comprising an insect material and the bacterium. Further, according to the present disclosure, it is also possible to provide a method for producing an animal in which the proportion of the bacterium in intestinal bacterial flora is controlled, a method for controlling the proportion of the bacterium in intestinal bacterial flora of an animal, and the like.

### Description of Embodiments

In the present specification, the terms "comprise" and "contain" include the concepts of "comprise," "contain," "consist essentially of," and "consist of."

### 1. Composition

The present disclosure provides as one embodiment a composition for controlling the proportion of a bacterium in intestinal bacterial flora, the composition comprising an insect material and the bacterium (also referred to herein as "the composition of the present disclosure"). This is explained below.

The insect material is not particularly limited, as long as it is an insect-derived material, i.e., a material prepared from an insect and containing substances, components, etc. that constitute the body of an insect.

The kind of insect is not particularly limited, as long as it is an insect species from which an insect material that has the above-prescribed calcium content and/or magnesium content can be prepared. Examples of insects include insects belonging to Diptera. Examples of insects belonging to Diptera include insects belonging to Brachycera, insects belonging to Nematocera, and the like; and are preferably insects belonging to Brachycera. Examples of insects belonging to Brachycera include insects belonging to Muscomorpha, insects belonging to Stratiomyomorpha, insects belonging to Tabanomorpha, insects belonging to Asilomorpha, insects belonging to Empidomorpha, and the like; and are preferably insects belonging to Muscomorpha, insects belonging to Stratiomyomorpha, and the like. Specific examples of insects belonging to Brachycera include insects belonging to Tephritidae, insects belonging to Muscidae, insects belonging to Stratiomyidae, insects belonging to Sarcophagidae, and the like; and are preferably insects belonging to Tephritidae, insects belonging to Muscidae, insects belonging to Stratiomyidae, and the like, more preferably insects belonging to Tephritidae, even more preferably insects belonging to *Ceratitis,* and particularly preferably *Ceratitis capitata.*

Usable insects are insects at any growth stage. Examples of insects include eggs, larvae, prepupae, pupae, and adult insects. These can be used entirely or partially.

Specific examples of insect materials include insect extracts, insect homogenates, and the like. The insect material may be one containing an insect body fluid and/or extraction solvent (a wet material), or one obtained by removing the insect body fluid and/or extraction solvent (a dry material). Regarding the properties thereof, the wet material is, for example, in the form of a liquid, a paste, or the like; and the dry material is, for example, in the form of a powder, particles, amorphous particles, or the like. Specific examples of forms of the insect material include concentrated solids, fat solids, reduced-fat solids, and the like.

Such insect materials can be used singly or in a combination of two or more.

Methods for producing insect materials are common, and would be apparent to one of ordinary skill in the art. An example of such a method for producing insect materials includes wet-milling of at least one whole insect, at least one whole adult insect, or at least one whole live insect into an insect slurry; and drying the insect slurry to form a dried insect material comprising solid insect matter particles. The drying may include spray drying or drum drying. As used herein, "insect slurry" is a semiliquid mixture of ground non-dewatered insects with or without added liquid. The semiliquid mixture includes a predetermined ratio of insects (mass) to added liquid (mass), such as water. For example, an insect slurry includes a 1:2 weight ratio of insects to water (e.g., 200 pounds (approx. 90.7 kg) of insects and 400 pounds (approx. 181.4 kg) of water). The step of drying may include heat drying, spray drying, freeze drying, tray drying, paddle drying, fluid bed drying, flash drying, air drying, vacuum drying, drum drying, or a combination thereof. Dryers, such as ovens, impingement ovens, freeze dryers, heat dryers, or hot air or vacuum dryers may be used. Spray drying is used to dry the insect slurry into a dried insect material. Drum drying is used to dry the insect slurry into a dried insect material. As used herein, "dried insect material" refers to an insect material (e.g., powder, flakes, meal) comprising water in an amount of about 15% by weight or less of the dried insect product. For example, the dried insect material comprises water in an amount of less than about 10% by weight.

Methods for obtaining whole insect or reduced-fat insect powder would be apparent to one of ordinary skill in the art. These methods can include non-limiting steps, such as blanching, wet milling, oil separation, and drying to obtain reduced-fat insect powder; or blanching, drying, and milling to obtain whole insect powder.

The advantages of wet milling include, for example:
(1) reducing the heat needed to preserve and dry the insect slurry;
(2) minimizing/avoiding the negative effects heat has on the insect material properties;
(3) increasing process efficiency;
(4) improving product digestibility; and
(5) improving process throughputs.

The wet-milling process may be performed using any one of, or a combination of, the following devices: grinders, mills, stone mills, mixers, peanut grinders, peanut butter grinders, colloid mills, pin mills, bead mills, dispersers, homogenizers, choppers, rotor stator devices, hammer mills, press grinders, mashers, macerators, food processors, rollers, or juicers. Examples of grinders include, but are not limited to, Olde Tyme Peanut Mill, Olde Tyme Peanut Grinder, Olde Tyme Peanut Butter Grinder, Olde Tyme Peanut Butter Mill, Old Style Peanut Mill, Olde Style Peanut Grinder, Olde Style Peanut Butter Grinder, Olde Style Peanut Butter Mill, IKA Mixer, IKA Colloid Mill, Jack LaLanne Power Juicer, or an Acme 6001 Centrifugal Juicer.

The content of the insect material in the composition of the present disclosure is not particularly limited, as long as it is an amount that can exhibit the desired effect. The content of the insect material is, for example, 0.1 mass% or more and less than 100 mass% based on 100 mass% of the composition of the present disclosure. When the composition of the present disclosure is a feed additive, described later, the content of the insect material can be, for example, 10 mass% or more and less than 100 mass%, 30 mass% or more and less than 100 mass%, 50 mass% or more and less than 100 mass%, 70 mass% or more and less than 100 mass%, 80 mass% or more and less than 100 mass%, or 90 mass% or more and less than 100 mass%. When the composition of the present disclosure is a feed, described later, the content of the insect material can be, for example, 0.1 mass% or more and 70 mass% or less, preferably 1 mass% or more and 50 mass% or less, more preferably 2 mass% or more and 30 mass% or less, and even more preferably 3 mass% or more and 20 mass% or less.

The composition of the present disclosure contains a bacterium that is the control target for its application (hereinafter also referred to as "the bacterium to be controlled"). The bacterium to be controlled can be a specific species of bacterium, several species of bacteria, whole bacteria from a taxon of several species of bacteria (e.g., genus and family), or whole bacteria from multiple taxa. The bacterium to be controlled is not particularly limited, as long as it can live in the intestine of the target organism of the composition of the present disclosure. Therefore, the bacterium to be controlled can be an intestinal bacterium. In one embodiment of the present disclosure, the bacterium to be controlled can be a so-called good bacterium, lactic acid bacterium (e.g., *Lactobacillus*, *Pediococcus, Tetragenococcus, Carnobacterium, Vagococcus, Leuconostoc, Weissella, Oenococcus, Atopobium, Streptococcus, Enterococcus, or Lactococcus* bacterium), *Bacillus subtilis* (*Bacillus* bacterium, *Bacillus subtilis* var. *natto,* or the like), butyric acid bacterium, or the like.

Examples of the bacterium to be controlled include bacteria of Firmicutes (organisms belonging to A (taxon name) are also referred to herein as "A organisms"), bacteria of Bacteroidetes, bacteria of Proteobacteria, bacteria of Actinobacteria, and the like; and preferably bacteria of Firmicutes, bacteria of Actinobacteria, and the like.

Examples of bacteria of Firmicutes preferably include bacteria of the class Bacilli, more preferably bacteria of the order Lactobacillales and bacteria of the order Bacillales. Examples of bacteria of the order Lactobacillales include bacteria of the family Lactobacillaceae (preferably *Lactobacillus* bacteria, *Pediococcus* bacteria, etc.), bacteria of the family Enterococcaceae (preferably *Enterococcus* bacteria etc.), bacteria of the family Streptococcaceae (preferably *Lactococcus* bacteria, *Streptococcus* bacteria, etc.), bacteria of the family Leuconostocaceae (preferably *Leuconostoc* bacteria etc.), bacteria of the family Aerococcaceae, bacteria of the family Carnobacteriaceae, and the like. Examples of bacteria of the order Bacillales include bacteria of the family Bacillaceae, preferably *Bacillus* bacteria, more preferably *Bacillus subtilis,* and even more preferably *Bacillus subtilis* var. *natto*, and the like.

Examples of Actinomycetota preferably include bacteria of the phylum Actinobacteria, more preferably bacteria of the order Bifidobacteriales. Examples of bacteria of the order Bifidobacteriales include bacteria of the family Bifidobacteriaceae, and preferably bacteria of the genus *Bifidobacterium* and the like.

The origin of the bacterium to be controlled contained in the composition of the present disclosure is not particularly limited. The bacterium to be controlled may be a bacterium adhering to the insect material, or a bacterium added separately from the insect material.

When the bacterium adhering to insect material is used as the bacterium to be controlled, as an embodiment of the present disclosure, the insect material (the bacterium adhering thereto) can be cultured in a suitable medium, thereby increasing the amount of the desired bacterium to be controlled. The bacterium adhering to the insect material used can be identified by analyzing the flora after the culture.

The medium contains a nutritional component generally used for the growth of bacteria. Examples of the nutritional component include carbon sources, nitrogen sources, sulfur sources, amino acids, inorganic salts, growth factors, and the like. The medium can be a synthetic medium, a semi-synthetic medium, a natural medium, or the like, according to the type of nutritional component etc. More specific examples of the nutritional component include peptone (e.g., casein peptone, meat peptone, heart muscle peptone, gelatin peptone, and soy peptone), extracts (e.g., yeast extract, meat extract, fish extract, heart extract, malt extract, potato extract, and tomato juice); sugars, such as glycerol, glucose, fructose, sucrose, molasses, and starch hydrolysate; organic acids, such as fumaric acid, citric acid, and succinic acid; amino acids or derivatives thereof, such as alanine, arginine, asparagine, cysteine, cystine, glutamic acid, glutamine, glycine, histidine, isoleucine, leucine, lysine, methionine, phenylalanine, proline, hydroxyproline, serine, threonine, tryptophan, tyrosine, and valine; inorganic ammonium salts, such as ammonium sulfate, ammonium chloride, and ammonium phosphate; organic nitrogen sources, such as soybean hydrolysates; inorganic nitrogen sources, such as ammonia gas and ammonia water; bases, such as adenine, guanine, and uracil; inorganic salts, such as sodium chloride, potassium chloride, calcium chloride, sodium phosphate, potassium phosphate, sodium carbonate, potassium carbonate, magnesium sulfate, iron ions (iron sulfate, iron nitrate, etc.), and manganese ions (manganese sulfate); various vitamins; and the like.

The medium is preferably a medium that can selectively grow the desired bacterium to be controlled (i.e., at a higher growth rate than other bacteria). Examples of such media include media for lactic acid bacteria. Various commercially available products can be used, and the above nutritional components can be added, as necessary, for use in culture.

The culture conditions are not particularly limited, and can be appropriately set according to the bacterium to be controlled. The culture temperature can be, for example, 0 to 45°C, 5 to 42°C, 15 to 40°C, or 25 to 35°C. The culture period can be, for example, 4 hours to 30 days, 8 hours to 20 days, 1 to 14 days, or 4 to 10 days. The culture atmosphere is either aerobic or anaerobic. The culture can be static culture or agitation culture.

When the bacterium added separately from the insect material is used as the bacterium to be controlled, a bacterium cultured as described above can be used, as necessary.

The total content of the insect material and the bacterium to be controlled in the composition of the present disclosure is not particularly limited, as long as it is an amount that can exhibit the desired effect. The total content is, for example, 0.1 mass% or more and 100 mass% or less based on 100 mass% of the composition of the present disclosure. When the composition of the present disclosure is a feed additive, described later, the total content can be, for example, 10 mass% or more and 100 mass% or less, 30 mass% or more and 100 mass% or less, 50 mass% or more and 100 mass% or less, 70 mass% or more and 100 mass% or less, 80 mass% or more and 100 mass% or less, or 90 mass% or more and 100 mass% or less. When the composition of the present disclosure is a feed, described later, the total content can be, for example, 0.1 mass% or more and 70 mass% or less, preferably 1 mass% or more and 50 mass% or less, more preferably 2 mass% or more and 30 mass% or less, and even more preferably 3 mass% or more and 20 mass% or less.

The occupancy of the bacterium to be controlled is, for example, 0.1% or more, preferably 0.2% or more, more preferably 0.5% or more, even more preferably 1% or more, still even more preferably 1.5% or more, and particularly preferably 2% or more, based on 100% of the total number of bacteria contained in the insect material (A: 0 when the insect material is sterilized) and bacteria including the bacterium to be controlled added separately from the insect material (B: 0 when no bacterium is added separately from the insect material) (A and B cannot both be 0). The upper limit of the occupancy is not particularly limited, and can be, for example, 100%% or 99.5%. The occupancy can be measured by or according to the method described in Example 1-1 below.

The composition of the present disclosure is not particularly limited, but is typically a feed additive or feed.

The composition of the present disclosure may contain fish meal, in addition to the above ingredients, if necessary. The fish meal is not particularly limited, and fish meal obtained from various kinds of raw material fish can be used. Examples of raw material fish include sardine, Alaska pollack (*Theragra chalcogramma*), saury, herring, catfish, bluegill, black bass, silver carp (*Hypophthalmichthys molitrix*), and the like. The fish meal can be obtained, for example, by heat-treating the raw material, and then drying and crushing the material. Such fish meal can be used singly or in a combination of two or more.

When the composition of the present disclosure contains fish meal, the content of the fish meal in the composition of the present disclosure is not particularly limited. For example, the content of the fish meal is in the range of 0.1 to 70 mass%, preferably 1 to 60 mass%, more preferably 0.5 to 50 mass%, and even more preferably 10 to 40 mass%. In some embodiments, the content of the fish meal is, for example, within a range in which the lower limit is any one of the following values, or the upper limit and the lower limit are any two of the following values: 0.1 mass%, 0.2 mass%, 0.5 mass%, 0.8 mass%, 1 mass%, 2 mass%, 5 mass%, 8 mass%, 10 mass%, 12 mass%, 15 mass%, 18 mass%, 20 mass%, 22 mass%, 25 mass%, 28 mass%, 30 mass%, 32 mass%, 35 mass%, 40 mass%, 50 mass%, and 60 mass%.

The composition of the present disclosure may contain a fish meal alternative, in addition to the above ingredients, if necessary. The fish meal alternative is not particularly limited. For example, protein-containing raw materials obtained from organisms other than fish (plants or animals) can be used. Specific examples of fish meal alternatives include plant feed ingredients such as soybean meal, soy protein concentrate, corn gluten meal, distiller's dried grains with solubles, and rapeseed oil meal; animal feed ingredients such as meat meal, meat bone meal, feather meal, and blood meal; microalgae ingredients; and single-cell organism raw materials such as yeast and bacteria. Such fish meal alternatives can be used singly or in a combination of two or more.

When the composition of the present disclosure contains a fish meal alternative, the content of the fish meal alternative in the composition of the present disclosure is not particularly limited. For example, the content of the fish meal alternative is in the range of 0.1 to 70 mass%, preferably 1 to 60 mass%, more preferably 5 to 50 mass%, and even more preferably 10 to 50 mass%. In some embodiments, the content of the fish meal alternative is, for example, within a range in which the lower limit is any one of the following values, or the upper limit and the lower limit are any two of the following values: 0.1 mass%, 0.2 mass%, 0.5 mass%, 0.8 mass%, 1 mass%, 2 mass%, 5 mass%, 8 mass%, 10 mass%, 12 mass%, 15 mass%, 18 mass%, 20 mass%, 22 mass%, 25 mass%, 28 mass%, 30 mass%, 32 mass%, 35 mass%, 40 mass%, 50 mass%, and 60 mass%.

The composition of the present disclosure may contain other ingredients, in addition to the above ingredients, if necessary. Examples of such other ingredients include binders (e.g., sodium carboxymethylcellulose (CMC), guar gum, sodium alginate, sodium polyacrylate, casein sodium, and propylene glycol), cereal powders (e.g., wheat flour and starch), minerals, vitamins (e.g., vitamin C, vitamin B1, vitamin A, vitamin D, and vitamin E), amino acids (e.g., lysine, methionine, and histidine), pigments (e.g., β-carotene, astaxanthin, and canthaxanthin), liver oils (e.g., cod liver oil), and the like.

When the composition of the present disclosure contains other ingredients, the content of other ingredients in the composition of the present disclosure is not particularly limited. For example, the content of other ingredients is 0.01 to 30 mass%.

The form of the composition of the present disclosure is not particularly limited, as long as it can be ingested (particularly orally) by the target animal, or it can be added when producing the composition in that form. That is, the composition of the present disclosure may be in any form such as a powder, granules, crumbles, pellets, cubes, a paste, or a liquid. For example, the composition of the present disclosure may be formed into pellets, such as dry pellets or moist pellets. The form of the composition of the present disclosure can be appropriately selected according to various conditions, such as the type and growth stage of the target animal. For example, when the target animal is an aquatic animal, for fry, the feed in the form of a powder or crumbles can be usually preferably used. For adult fish, dry pellets can be usually preferably used.

The use of the composition of the present disclosure is to control the proportion of the bacterium to be controlled in intestinal bacterial flora, and is not particularly limited in this respect. The composition of the present disclosure can increase the proportion of the bacterium to be controlled in intestinal bacterial flora. For example, according to the composition of the present disclosure, the proportion of the bacterium to be controlled in intestinal bacterial flora can be increased by, for example, 1% or more, 2% or more, 3% or more, 4% or more, 5% or more, 6% or more, 7% or more, 8% or more, 9% or more, 10% or more, 12% or more, 15% or more, 17% or more, or 20% or more. The proportion of the bacterium to be controlled in intestinal bacterial flora is the occupancy of the bacterium to be controlled relative to 100% of the total bacteria count of the intestinal bacterial flora, and can be measured by or according to the method described in Example 1-2 or 2-2 below.

The intestinal bacterial flora is the intestinal bacterial flora of the target animal of the composition of the present disclosure. The target animal is not particularly limited, as long as the animal has an intestine in which bacteria can live. Preferred examples of the target animal include aquatic animals, and other examples include various mammals (e.g., humans, monkeys, mice, rats, dogs, cats, rabbits, pigs, horses, cows, sheep, goats, and deer), avian animals, amphibian animals, reptile animals, and the like. The aquatic animal is not particularly limited, as long as the animal can live in water. Examples of aquatic animals include fish, crustaceans, shellfish, and the like. Among these, fish are preferable. Specific examples of fish include red seabream (*Pagrus major*), carp, trout, yellowtail (*Seriola quinqueradiata*), young yellowtail, greater yellowtail (*Seriola dumerili*), yellowtail amberjack (*Seriola lalandi*), tuna, puffer fish, bastard halibut (*Paralichthys olivaceus*), horse mackerel, mackerel, grouper, *Epinephelus bruneus,* salmon, eel, catfish (*Silurus asotus*), *Plecoglossus altivelis altivelis,* char (*Salvelinus pluvius*)*,* eel, carp, sea bass, crucian carp, trout, *Oncorhynchus mason,* lake smelt (*Hypomesus nipponensis*), goldfish, killifish (*Oryzias latipes*), tilapia (*Oreochromis niloticus*), sturgeon, zebrafish (*Danio rerio*), and the like. Examples of crustaceans include shrimp (prawns) and crabs. Specific examples of crustaceans include vannamei shrimp (*Litopenaeus vannamei*), Japanese tiger prawn (*Marsupenaeus japonicus*), black tiger prawn (*Penaeus monodon*), white leg shrimp (*Litopenaeus vannamei*), and swimming crab *(Portunus trituberculatus).*

### 2. Production Method and Control Method

The present disclosure provides as one embodiment a method for producing an animal in which the proportion of a bacterium in intestinal bacterial flora is controlled, the method comprising feeding the animal the composition of the present disclosure, and a method for controlling the proportion of a bacterium in intestinal bacterial flora of an animal, the method comprising feeding the animal the composition of the present disclosure. These are explained below.

The feeding amount of the composition of the present disclosure is not particularly limited. The feeding amount of the composition of the present disclosure can be appropriately selected according to various conditions, such as the type of target aquatic animal. The feeding amount of the composition of the present disclosure can be, for example, the amount of satiation. The composition of the present disclosure can be fed once a day, or two or more times a day in divided portions. Alternatively, the composition of the present disclosure can be fed once every several days. The feeding amount of the composition of the present disclosure at the time of each feeding may or may not be constant.

The period of feeding of the composition of the present disclosure is not particularly limited and can be appropriately selected according to various conditions, such as the type of target animal. The composition of the present disclosure can be continuously fed over the entire period of cultivation (breeding), or may be fed only in a partial period of cultivation (breeding). The term "partial period" may refer to, for example, a period consisting of 10% or more, 20% or more, 30% or more, 50% or more, 70% or more, or 90% or more of the entire period of cultivation (breeding). The period of feeding of the composition of the present invention can be, for example, 3 days or longer, 1 week or longer, 2 weeks or longer, 3 weeks or longer, 4 weeks or longer, 2 months or longer, or 3 months or longer; can be 1 year or shorter, 6 months or shorter, 4 months or shorter, 3 months or shorter, 2 months or shorter, 4 weeks or shorter, or 3 weeks or shorter; or can be within a range defined by any consistent combination of these ranges. The feeding of the composition of the present invention may be continued and interrupted repeatedly for arbitrary periods.

Cultivation (breeding) of the animal can be performed in the same manner as usual methods for cultivating (breeding) an animal, except that the composition of the present disclosure is fed. For example, cultivation (breeding) of an aquatic animal can be performed, for example, in a fish pen in the sea, or a fish tank on land. The term "cultivation" includes both propagation cultivation, in which only seed organisms are produced (fry production etc.), and cultivation in which fish are raised to produce adult organisms (parent fish etc.).

### Examples

The present invention is described in detail below based on Examples; however, the present invention is not limited by these Examples.

### Reference Example 1

An insect powder was produced as described below. The insect powder (reduced-fat protein powder and whole larvae powder) was produced using *Ceratitis capitata* larvae as raw materials. Specifically, the reduced-fat protein powder was obtained as follows: Larvae were washed, blanched at 92°C for 6 minutes, and then crushed with a stone mill; the obtained composition was centrifuged; and a precipitate was collected, dried, and crushed. The whole larvae powder was specifically obtained by washing and blanching larvae at 92°C for 6 minutes, and then performing drying and crushing. In the following Examples, the reduced-fat protein powder was used as the insect powder.

### Example 1

### Example 1-1: Measurement 1 of Bacteria Adhering to Insect Powder

100 ml of MRS medium and 0.6 g of the insect powder (Reference Example 1) were placed in a baffled flask, and static culture was performed at 30°C under anaerobic conditions. After 7 days, the medium was centrifuged at 1000 rpm for 10 minutes to obtain an insect powder culture as a precipitate. The composition of the MRS medium is as shown in Table 1.

**Table 1**

| | |
|---|---|
| Difco lactobacilli MRS Broth | 55gIL |
| Proteose Peptone No. 3 | 10g/L |
| Beef extract | 10g/L |
| Yeast extract | 5g/L |
| Dextrose | 20g/L |
| Polysorbate 80 | 1g/L |
| Ammonium Citrate | 2g/L |
| Sodium Acetate | 5g/L |
| Magnesium Sulfate | 0.1g/L |
| Manganese Sulfate | 0.05g/L |
| Dipotassium Phosphate | 2g/L |
| pH 6.5 | |

DNA in the insect powder culture was purified, the 16S rRNA V1-V2 region was amplified, and sequence analysis was performed using a next-generation sequencer MiSeq (Illumina). After quality filtering, 10,000 reads were randomly selected for each sample, clustered with 97% homology, and classified into operational taxonomic units (OTUs). The representative sequences of the formed OTUs were collated with the full-length 16S gene database, and species identification and composition analysis were performed. For flora composition analysis from clustering, the pipeline of QIIME (Nat Methods. 2010 May; 7(5): 335-6. doi: 10.1038/nmeth.f.303. Epub 2010 Apr 11.) was used, and Geengenes (Applied and Environmental Microbiology, July 2006, Vol. 72, No. 7, pp. 5069-5072, doi: 10.1128/AEM.03006-05) was used as the full-length 16S gene database.

As a result of measuring the flora of the insect powder culture, *Vagococcus* bacteria occupied 2.3% of the total number of bacteria in the insect powder culture, which was taken as 100%.

### Example 1-2: Feeding 1 for Red Seabream

As test diets, a fish meal diet containing fish meal as the main protein source (FM), a diet in which part of fish meal was replaced with a soy protein concentrate (LFM), and a diet in which 50% of the fish meal contained in LFM was replaced with an insect powder (from the same production batch of the one used in Example 1-1) (FFM) were produced in the form of pellets (diameter: 3.2 mm, length: 3 to 5 mm). The compositions of FM, LFM, and FFM are shown in Table 2. In Table 2, the numerical values are in parts by mass.

**Table 2**

| | FM | LFM | FFM |
|---|---|---|---|
| Fish meal | 500 | 300 | 150 |
| Soy protein concentrate | 0 | 210 | 210 |
| Insect powder | 0 | 0 | 150 |
| Soybean meal | 200 | 200 | 200 |
| Com qluten meal | 100 | 100 | 100 |
| Wheat flour | 70 | 32 | 28 |
| DL-methionine | 0 | 2 | 3 |
| Lysine | 0 | 1 | 2 |
| Taurine | 0 | 5 | 5 |
| Vitamin | 20 | 20 | 20 |
| Mineral | 20 | 20 | 20 |
| Guar gum | 5 | 5 | 5 |
| CMC | 20 | 20 | 20 |
| Cod liver oil | 65 | 85 | 87 |
| Total | 1000 | 1000 | 1000 |

Ten red seabreams with an average fish weight of 58 g were housed in each 1.1-ton tank. Three tanks were provided for each of the FM test group and the FFM test group, for a total of nine tanks. Each test diet was fed once a day for 7 days a week until satiation. After one week of feeding, five red seabreams were taken out at random and anesthetized, then the abdomen was pressed, and the feces were collected.

DNA in the feces was purified, the 16S rRNA V1-V2 region was amplified, and sequence analysis was performed using a next-generation sequencer MiSeq (Illumina). After quality filtering, 10,000 reads were randomly selected for each sample, clustered with 97% homology, and classified into operational taxonomic units (OTUs). The representative sequences of the formed OTUs were collated with the full-length 16S gene database, and species identification and composition analysis were performed. For flora composition analysis from clustering, the pipeline of QIIME was used, and Geengenes was used as the full-length 16S gene database.

The results are shown in Table 3. The occupancy of *Vagococcus* bacteria in the intestine of red seabreams fed with the diet containing the insect powder was higher than in the fish not fed with the insect powder. It was considered that *Vagococcus* bacteria adhering to the insect powder entered the intestine of fish and increased the occupancy in the intestine.

**Table 3**

| Test group | FM | | | | |
|---|---|---|---|---|---|
| Red seabream ID | 1 | 2 | 3 | 4 | 5 |
| Occupancy of *Vagococcus* bacteria (%) | 0 | 0 | 0 | 0 | 0 |

| Test group | LFM | | | | |
|---|---|---|---|---|---|
| Red seabream ID | 6 | 7 | 8 | 9 | 10 |
| Occupancy of *Vagococcus* bacteria (%) | 0 | 0 | 0 | 0 | 0 |

| Test group | FFM | | | | |
|---|---|---|---|---|---|
| Red seabream ID | 11 | 12 | 13 | 14 | 15 |
| Occupancy of *Vagococcus* bacteria (%) | 20.5 | 29.5 | 20.4 | 22.4 | 23.1 |

### Example 2

### Example 2-1: Measurement 2 of Bacteria Adhering to Insect Powder

Using an insect powder from a different production batch from that of the insect powder used in Example 1-1, bacteria adhering to the insect powder were cultured and measured in the same manner as in Example 1-1-. As a result of measuring the flora of the insect powder culture, Bacillaceae bacteria occupied 99.3% of the total number of bacteria in the insect powder culture, which was taken as 100%.

### Example 2-2: Feeding 2 for Red Seabream

As test diets, a fish meal diet containing fish meal as the main protein source (FM) and a diet containing a small amount of an insect powder (from the same production batch of the one used in Example 2-1) (FFM) were produced in the form of pellets (diameter: 3.2 mm, length: 3 to 5 mm). The compositions of FM and FFM are shown in Table 4. In Table 4, the numerical values are in parts by mass.

**Tale 4**

| | FM | FFM |
|---|---|---|
| Fish meal | 550 | 560 |
| Insect powder | | 50 |
| Soybean meal | 80 | 80 |
| Com qluten meal | 80 | 80 |
| Flour | 100 | 92 |
| α-Cellulose | 57 | 0 |
| Vitamin | 20 | 20 |
| Mineral | 20 | 20 |
| Guar qum | 5 | 5 |
| CMC | 20 | 20 |
| Cod liver oil | 68 | 73 |
| Total | 1000 | 1000 |

Ten red seabreams with an average fish weight of 46 g were housed in each 1.1-ton tank. Three tanks were provided for each of the FM test group and the FFM test group, for a total of six tanks. Each test diet was fed once a day for 7 days a week until satiation. After one week of feeding, three red seabreams were taken out at random from each tank and anesthetized, then the abdomen was pressed, and the excreted feces were collected. The DNA in the feces was purified, and species identification, composition analysis, and flora composition analysis were performed in the same manner as in Example 1-2.

The results are shown in Table 5. The occupancy of Bacillaceae bacteria in the intestine of red seabreams fed with the diet containing the insect powder was higher than in the fish not fed with the insect powder. It was considered that Bacillaceae bacteria adhering to the insect powder entered the intestine of fish and increased the occupancy in the intestine.

**Table 5**

| Test group | FM | | | | | | | | |
|---|---|---|---|---|---|---|---|---|---|
| Red seabream ID | 1 | 2 | 3 | 4 | 5 | 6 | 7 | 8 | 9 |
| Occupancy of Bacillaceae bacteria (%) | 0 | 0 | 0 | 0 | 0 | 0 | 0 | 0 | 0 |

| Test group | FFM | | | | | | | | |
|---|---|---|---|---|---|---|---|---|---|
| Red seabream ID | 10 | 11 | 12 | 13 | 14 | 15 | 16 | 17 | 18 |
| Occupancy of Bacillaceae bacteria (%) | 44.6 | 32.2 | 25.3 | 32.7 | 23.9 | 3.7 | 24.5 | 11.1 | 22.2 |

### Test Example 1: Collection of Fish Feces

As a test diet, a diet containing a small amount of the insect powder (FFM) can be produced in the form of pellets (diameter: 3.2 mm, length: 3 to 5 mm). FFM requires proper regulation of crude protein and crude lipid levels for fish growth, and red seabream is required to have 400 g or more of crude protein and 100 g or more of crude lipid. After continuing feeding for 2 weeks or more, the feces are collected. The feces are collected by pressing the abdomen of the fish after anesthesia.

### Test Example 2: Methods for Isolation of Bacteria from Fish Feces and Growth Thereof

After diluting the fish feces with physiological saline, culture is performed using medium. Then, the grown colonies can be randomly separated and propagated on medium.

### Test Example 3: Method for Confirming Antibacterial Activity

The presence or absence of antibacterial activity against the indicator strain can be examined using a direct method (known method). Specifically, the isolated strains are spotted on LB agar medium with a toothpick and then aerobically cultured. Next, soft agar containing the indicator strain is layered on the spotted agar medium, followed by aerobic culture, and the antibacterial activity can be confirmed by the presence or absence of halo formation.

Various strains can be used as the indicator strain. Usable examples include *Aeromonas salmonicida,* atypical *Aeromonas salmonicida, Aeromonas hydrophila, Aeromonas caviae, Aeromonas sobria, Aeromonas jandaei, Aeromonas enteropelogenes, Enterococcus faecalis,* and the like.

### Test Example 4: Fish Infection Test

The disease resistance can be confirmed by infecting fish with bacteria, and comparing the survival rate of fish fed with a material containing insect powder and useful bacteria and those not fed with it.

## Claims

1. A composition for controlling the proportion of a bacterium in intestinal bacterial flora, the composition comprising an insect material and the bacterium.

2. The composition according to claim 1, wherein the bacterium is at least one member selected from the group consisting of bacteria of Firmicutes, bacteria of Bacteroidetes, bacteria of Proteobacteria, and bacteria of Actinobacteria.

3. The composition according to claim 1 or 2, wherein the insect material is a material derived from an insect belonging to Diptera.

4. The composition according to any one of claims 1 to 3, wherein the insect material is at least one member selected from the group consisting of insect extracts and insect homogenates.

5. The composition according to any one of claims 1 to 4, wherein the insect material is a material derived from at least one member selected from the group consisting of eggs, larvae, prepupae, pupae, and adult insects.

6. The composition according to any one of claims 1 to 5, wherein the intestinal bacterial flora is intestinal bacterial flora of an aquatic animal.

7. The composition according to claim 6, wherein the aquatic animal is a fish.

8. The composition according to any one of claims 1 to 7, wherein the content of the insect material is 1 to 50 mass%.

9. The composition according to any one of claims 1 to 8, which is a feed additive or a feed.

10. A method for producing an animal in which the proportion of a bacterium in intestinal bacterial flora is controlled, the method comprising feeding the animal the composition of any one of claims 1 to 9.

11. A method for controlling the proportion of a bacterium in intestinal bacterial flora of an animal, the method comprising feeding the animal the composition of any one of claims 1 to 9.

12. Use of an insect material and a bacterium for producing a composition for controlling the proportion of the bacterium in intestinal bacterial flora.

13. Use of a composition comprising an insect material and a bacterium for controlling the proportion of the bacterium in intestinal bacterial flora.

14. A composition comprising an insect material and a bacterium for use in control of the proportion of the bacterium in intestinal bacterial flora.
